# EUROPEAN PATENT APPLICATION

(11) **EP 1 298 145 A1**
(43) Date of publication of application: **02.04.2003**
(21) Application number: 01945629.2
(22) Date of filing: 27.06.2001
(51) Int. Cl.: C08B 37/02, C07D 491/22, A61K 47/48, A61K 31/4745, A61K 47/36, A61P 35/00

(54) **DDS COMPOUND AND PROCESS FOR THE PREPARATION THEREOF**

(30) Priority: 29.06.2000 JP 2000195919
(71) Applicant: DAIICHI PHARMACEUTICAL CO., LTD., Chuo-ku, Tokyo 103-8234 (JP)
(72) Inventor: IMURA, Akihiro, Edogawa-ku, Tokyo 134-8630 (JP); NOGUCHI, Shigeru, Edogawa-ku, Tokyo 134-8630 (JP); YAMAGUCHI, Tatsuya, Edogawa-ku, Tokyo 134-8630 (JP); YAGI, Tsutomu, Edogawa-ku, Tokyo 134-8630 (JP); KAWABE, Takefumi, Edogawa-ku, Tokyo 134-8630 (JP)
(74) Representative: Godemeyer, Thomas, Dr.
(86) International application number: JP0105498
(87) International publication number: WO02000734

(57) **Abstract**

A DDS compound in which amino group at 1-position of (1S,9S)-1-amino-9-ethyl-5-fluoro-2,3-dihydro-9-hydroxy-4-methyl-1H,12H-benzo[de]pyrano[3',4':6,7]-indolizino[1,2-b]quinoline-10,13(9H,15H)-dione as a drug compound is bound to a carboxyl group of a carboxymethyldextran polyalcohol with a spacer containing one amino acid or two to eight amino acids linked by peptide bond(s); characterized in that an introduced amount of residue of the drug compound is in a range of from 3.2% to 8.4% by weight; a weight-average molecular weight of the carboxymethyldextran polyalcohol is in a range of from 240,000 to 480,000; and degree of carboxymethylation is in a range of from 0.14 to 0.47; and a method for preparing said DDS compound, which comprises the steps of, for example, adding an aqueous solution containing sodium periodate to an aqueous solution containing dextran at a temperature of 4°C ± 2°C to oxidize the dextran, and then adding the resulting reaction mixture to an aqueous solution containing sodium borohydride at a temperature not higher than 15°C to obtain a dextran polyalcohol.

## Description

### Technical Field

The present invention relates to a compound for the drug delivery system (hereinafter referred to as "a DDS compound") in which a polysaccharide derivative obtained by polyalcoholizing carboxymethyldextran is bound to a drug compound, and a method for preparation thereof.

### Background Art

Antineoplastic agents, used for treatment of solid cancers such as lung cancer or digestive organ carcinomas and blood cancers such as leukemia, are systemically administered through routes of administration such as intravenous or oral administration, and then are distributed to certain tumorous sites and inhibit or suppress the proliferation of cancer cells to exhibit their therapeutic efficacy. However, the systemically-administered antineoplastic agents are rapidly taken into livers and reticuloendothelial organs from blood, or rapidly excreted into urine, and accordingly, their blood concentrations may sometimes be lowered to render the distribution into tumorous sites become insufficient. In addition, common antineoplastic agents themselves have poor distribution-selectivity to tumorous sites (tumor selectivity), and therefore, the antineoplastic agents are widely distributed over various tissues and cells of the whole body and act as cytotoxins also against normal cells and tissues, which results in problems of the appearance of adverse effects, e.g., diarrhea, pyrexia, emesis, or alopecia at an extremely high rate. Therefore, it has been desired to develop a means of efficiently and selectively distributing antineoplastic agents to tumorous sites.

As one of such means, a process has been proposed in which a polysaccharide derivative is used as a drug carrier, and an antineoplastic agent is bound to the polysaccharide derivative to delay the disappearance of the antineoplastic agent from blood and to enhance selectivity to tumor tissues. Already disclosed means are those in which a carboxyl group of a polysaccharide having carboxyl groups is bound to a drug with a peptide chain (International Publication WO094/19376); those in which a drug is introduced into a carboxymethylated mannoglucan derivative by means of a Schiff base or an acid amide bond (Japanese Patent Publication (KOKOKU) No. (Hei) 7-84481/1995); those in which a polyalcoholized polysaccharide derivative is used as a drug carrier and the derivative is bound to a drug with a peptide chain or a peptide chain and p-aminobenzyloxycarbonyl group (International Publication WO99/61061) and the like.

Among DDS compounds using a polysaccharide derivative as a drug carrier, those using a polysaccharide derivative as a drug carrier, in which carboxymethyldextran is polyalcoholized, and bound to a drug compound residue with a peptide chain have especially excellent tumor selectivity and are expected to be developed as antineoplastic agents. In particular, the DDS compounds bound to (1S,9S)-1-amino-9-ethyl-5-fluoro-2,3-dihydro-9-hydroxy-4-methyl-1H,12H-benzo[de]-pyrano[3',4':6,7]indolizino[1,2-b]quinoline-10,13(9H,15H)-dione as a drug compound residue can exert excellent tumor selectivity and antineoplastic activity and can be expected to be clinically useful.

However, studies by the inventors of the present invention revealed that safety and effective range of the aforementioned DDS compounds, in which (1S,9S)-1-amino-9-ethyl-5-fluoro-2,3-dihydro-9-hydroxy-4-methyl-1H,12H-benzo[de]pyrano[3',4':6,7]-indolizino[1,2-b]quinoline-10,13(9H,15H)-dione is bound as a drug compound residue with a peptide spacer to a polysaccharide derivative obtained by polyalcoholization of carboxymethyldextran, widely vary depending on the change of the molecular weight of the macromolecular carrier moiety as a drug carrier, the degree of carboxymethylation, and the introduced amount of the aforementioned drug compound residue. From this reason, it has been desired to choose a specific DDS compound having high safety and a broad effective range among the aforementioned DDS compounds.

The inventors of the present invention also encountered a problem that, in the preparation of a carboxymethyldextran polyalcohol, exothermic process of dextran polyalcohol preparation caused a decrease of the molecular weight of a macromolecular carrier, and exothermic process of carboxymethylation of the dextran polyalcohol gave insufficient control of the degree of carboxymethylation, thereby a macromolecular carrier with a constant quality was not obtainable. In addition, there are also problems that, in the step of binding the aforementioned drug compound to a peptide spacer and the step of binding the drug compound to a macromolecular carrier with a peptide spacer, conventional methods required separation and purification of the desired product, which made the operations troublesome, and the methods gave only a poor yield of a desired product and a product of good quality was not provided. These problems were desired to be solved.

### Disclosure of the Invention

Accordingly, an object of the present invention is to provide the aforementioned DDS compound wherein the molecular weight and the degree of carboxymethylation of the macromolecular carrier as a drug carrier and the introduced amount of the residue of the aforementioned drug compound is chosen to give high safety and a broad range of effectiveness. In addition, another object of the present invention is to provide a method of preparation which achieves efficient preparation of the aforementioned specific DDS compound with a high quality in a high yield, and is suitable for industrial application.

The inventors of the present invention conducted intensive studies to achieve the foregoing objects, and as a result, succeeded in choosing a compound having high safety and a broad range of effectiveness from the aforementioned DDS compounds. More specifically, the inventors carried out optimization of the molecular weight and the degree of carboxymethylation of the macromolecular carrier moiety as a drug carrier, and the introduced amount of the residue of the aforementioned drug compound, and found that the compound satisfying the specific conditions has high safety and a broad range of effectiveness. They also found that the desired DDS compound with constant quality can efficiently be prepared by choosing a means of controlling a reaction temperature, a means of monitoring the progress of the reaction, reagents and the like in the preparation of the aforementioned specific DDS compound. The present invention was achieved on the basis of these findings.

The present invention thus provides a DDS compound in which the amino group at the 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione is bound to a carboxyl group of a carboxymethyldextran polyalcohol with a spacer containing one amino acid or two to eight amino acids linked by peptide bond(s), characterized in that
(1) an introduced amount of the residue of (1S,9S)-1-amino-9-ethyl-5-fluoro-2,3-dihydro-9-hydroxy-4-methyl-lH,12H-benzo[de]pyrano[3',4':6,7]indolizino[1,2-b]-quinoline-10,13(9H,15H)-dione is in a range of from 3.2% to 8.4% by weight based on the total weight of the DDS compound;
(2) a weight-average molecular weight of the carboxymethyldextran polyalcohol based on pullulan standard is in a range of from 240,000 to 480,000; and
(3) degree of carboxymethylation of the carboxymethyldextran polyalcohol is in a range of from 0.23 to 0.47.

The present invention also provides a DDS compound in which the amino group at the 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione is bound to a carboxyl group of a carboxymethyldextran polyalcohol with a spacer containing one amino acid or two to eight amino acids linked by peptide bond(s), characterized in that
(1) an introduced amount of the residue of (1S,9S)-1-amino-9-ethyl-5-fluoro-2,3-dihydro-9-hydroxy-4-methyl-1H,12H-benzo[de]pyrano[3',4':6,7]indolizino[1,2-b]-quinoline-10,13(9H,15H)-dione is in a range of from 3.2% to 8.4% by weight based on the total weight of the DDS compound;
(2) a weight-average molecular weight of the carboxymethyldextran polyalcohol based on pullulan standard is in a range of from 240,000 to 480,000; and
(3) degree of carboxymethylation of the carboxymethyldextran polyalcohol is in a range of from 0.14 to 0.47. The present invention further provides the aforementioned DDS compound wherein the degree of carboxymethylation of the aforementioned carboxymethyldextran polyalcohol is measured by capillary electrophoresis using a calibration curve which is obtained by measuring a standard substance by decomposition method or NMR method.

Moreover, the present invention provides a medicament which comprises the aforementioned DDS compound and an antineoplastic agent which comprises the aforementioned DDS compound; and a use of the aforementioned DDS compound for the manufacture of the aforementioned medicament; and a method for therapeutic treatment of a malignant tumor which comprises the step of administering a therapeutically effective amount of the aforementioned DDS compound to a mammal including a human.

According to another aspect of the present invention, a method for preparing the aforementioned DDS compound is provided. The method of the present invention is a method for preparing the aforementioned DDS compound which comprises one or more steps selected from the group consisting of the following steps of:
(A) adding an aqueous solution containing sodium periodate to an aqueous solution containing dextran at a temperature of 4°C ± 2°C to oxidize the dextran, and then adding the resulting reaction mixture to an aqueous solution containing sodium borohydride at a temperature not higher than 15°C to obtain dextran polyalcohol;
(B) reacting a dextran polyalcohol with sodium monochloroacetate to prepare carboxymethyldextran polyalcohol, characterized in that the end of reaction for the carboxymethylation is determined by capillary electrophoresis;
(C) condensing the amino group at the 1-position of (1S,9S)-1-amino-9-ethyl-5-fluoro-2,3-dihydro-9-hydroxy-4-methyl-lH,12H-benzo[de]pyrano[3',4':6,7]-indolizino[1,2-b]quinoline-10,13(9H,15H)-dione with the α-carboxyl group of an amino acid whose α-amino group is protected with tert-butoxycarbonyl group, or with the C-terminal carboxyl group of an oligopeptide containing two to eight amino acids whose N-terminal is protected with tert-butoxycarbonyl group, characterized in that 1-ethyl-3-(dimethylaminopropyl)carbodiimide or a salt thereof is used as a condensing agent; and
(D) condensing with a carboxymethyldextran polyalcohol a deprotected compound obtained by eliminating tert-butoxycarbonyl group from a condensate in which the amino group at the 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10, 13(9H,15H)-dione is condensed with the α-carboxyl group of an amino acid whose a -amino group is protected with tert-butoxycarbonyl group, or with the C-terminal carboxyl group of an oligopeptide containing two to eight amino acids whose N-terminal is protected with tert-butoxycarbonyl group, characterized in that 1-ethyl-3-(dimethylaminopropyl)carbodiimide or a salt thereof is used as a condensing agent.

A preferred method of the present invention comprises two or more steps selected from the aforementioned steps of (A) to (D), a more preferred method comprises three or more steps selected from the aforementioned steps of (A) to (D), and a particularly preferred method comprises all of the aforementioned steps of (A) to (D). As a preferred embodiment, provided is the aforementioned method wherein the end of the condensation is determined by high-performance liquid chromatography in step (D).

The present invention further provides a carboxymethyldextran polyalcohol used for the preparation of the aforementioned DDS compound whose weight-average molecular weight based on pullulan standard is in a range of from 240,000 to 480,000 and degree of carboxymethylation is in a range of from 0.23 to 0.47; a carboxymethyldextran polyalcohol used for the preparation of the aforementioned DDS compound whose weight-average molecular weight by the pullulan standard is in a range of from 240,000 to 480,000 and degree of carboxymethylation is in a range of from 0.14 to 0.47; and the aforementioned carboxymethyldextran polyalcohol wherein the degree of carboxymethylation thereof is measured by the capillary electrophoresis using a calibration curve which is obtained by the decomposition method or the NMR method.

In addition, the present invention provides a use of the aforementioned carboxymethyldextran polyalcohol for the preparation of the aforementioned DDS compound.

Moreover, the present invention provides a method for preparing the aforementioned carboxymethyldextran polyalcohol, which comprises the steps of:
(A) adding an aqueous solution containing sodium periodate to an aqueous solution containing dextran at a temperature of 4°C ± 2°C to oxidize the dextran, and then adding the resulting reaction mixture to an aqueous solution containing sodium borohydride at a temperature not higher than 15°C to obtain a dextran polyalcohol; and
(B) reacting the dextran polyalcohol obtained in the step (A) with sodium monochloroacetate to prepare the carboxymethyldextran polyalcohol, characterized in that the end of reaction for the carboxymethylation is determined by the capillary electrophoresis.

### Best Mode for Carrying out the Invention

The entire disclosure in Japanese Patent Application No. 2000-195919 (filed on June 29, 2000) is incorporated by reference in the disclosure of the present specification.

The DDS compound of the present invention is a DDS compound wherein amino group at the 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione (hereinafter sometimes referred to as "the drug compound") is bound to a carboxyl group of a carboxymethyldextran polyalcohol with a spacer containing one amino acid or two to eight amino acids linked by peptide bond(s), characterized in that
(1) the introduced amount of the residue of the aforementioned drug compound is in a range of from 3.2% to 8.4%, preferably from 5.6% to 7.6% by weight based on the total weight of the DDS compound;
(2) the weight-average molecular weight of the carboxymethyldextran polyalcohol based on the pullulan standard is in a range of from 240,000 to 480,000; and
(3) the degree of carboxymethylation of the carboxymethyldextran polyalcohol is in a range of from 0.23 to 0.47.

Another DDS compound provided by the present invention is a DDS compound wherein the amino group at the 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]-quinoline-10,13(9H,15H)-dione is bound to a carboxyl group of a carboxymethyldextran polyalcohol with a spacer containing one amino acid or two to eight amino acids linked by peptide bond(s), characterized in that
(1) the introduced amount of the residue of the aforementioned drug compound is in a range of from 3.2% to 8.4%, preferably from 5.6% to 7.6% by weight of the total weight of the DDS compound;
(2) the weight-average molecular weight of the carboxymethyldextran polyalcohol by the pullulan standard is in a range of from 240,000 to 480,000; and
(3) the carboxymethylation degree of the aforementioned carboxymethyldextran polyalcohol is in a range of from 0.14 to 0.47.

In the specification, numerical ranges represented by "from --- to" are ranges including numerical values of the lower and upper limits.

International Publication WO97/46260 discloses a drug complex in which the amino group at the 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10, 13(9H,15H)-dione is bound to a carboxyl group of a carboxymethyldextran polyalcohol with a spacer containing one amino acid or two to eight amino acids linked by peptide bond(s). However, the aforementioned specific DDS compound is not disclosed.

In the DDS compound of the present invention, the weight-average molecular weight of the carboxymethyldextran polyalcohol which functions as a drug carrier ranges from 240,000 to 480,000. The weight-average molecular weight of the carboxymethyldextran polyalcohol based on the pullulan standard can be determined by a method well-known in the art, for example, according to the method of gel filtration chromatography using pullulan as a standard. Pullulan used as the standard is commercially available from Shodex Co. and the like. The degree of carboxymethylation of the carboxymethyldextran polyalcohol is in a range of from 0.14 to 0.47, or from 0.23 to 0.47.

The degree of carboxymethylation of the carboxymethyldextran polyalcohol can be measured according to a method well-known in the art, for example, according to the method of capillary electrophoresis. When the degree of carboxymethylation of the carboxymethyldextran polyalcohol is measured by the capillary electrophoresis, a calibration curve obtained by using a standard substance can be used. As the standard substances, several kinds of carboxymethyldextran polyalcohol having different introduced amounts of carboxymethyl group can be prepared and used. The calibration curve may be obtained by either a decomposition method or an NMR method. The decomposition method and the NMR method may sometimes give different measured values of the degree of carboxymethylation for the same standard substance. In general, a value of the degree of carboxymethylation determined by the NMR method tends to be lower by approximately 0.09 compared to a value determined by the decomposition method. Accordingly, when a calibration curve obtained by the NMR method is used, the degree of carboxymethylation is desirably in a range of from 0.14 to 0.38.

In the decomposition method, glycerol (Glr), glycolaldehyde (GA), carboxymethylglycerol (CM-Glr), and carboxymethylglycolaldehyde (CM-GA) are determined, each of which is quantitatively generated by acid hydrolysis of the carboxymethyldextran polyalcohol. Glycerol in the hydrolysate can directly be determined under basic conditions by using high-performance liquid chromatography, and glycolaldehyde can be determined by reacting the same with dansylhydrazine as an aldehyde labeling reagent and then subjecting the reaction product to high-performance liquid chromatography. Carboxymethylglycerol and carboxymethylglycolaldehyde can be determined by reducing the aldehyde group of carboxymethylglycolaldehyde to convert the same into carboxymethylethylene glycol (CM-EG), then reacting respectively with 9-anthryldiazomethane as a fluorescent labeling reagent of carboxylic acid, and subjecting the reaction products to high-performance liquid chromatography. The degree of carboxymethylation can be calculated from the following formula:
CM-Glr/(Glr+CM-Glr)+CM-EG/(GA+CM-EG).

In the NMR method, several kinds of carboxymethyldextran polyalcohol having different introduced amounts of carboxymethyl group are used as standard substances, and ¹³C-NMR thereof was measured. The integrated intensity of each of four signals is calculated at the C-1 position and the C-5 position of the carboxymethyldextran polyalcohol of each standard substance and at the C-1 position and the C-5 position at which carboxymethyl group is bound to the side chain moiety, and the degree of carboxymethylation of each carboxymethyldextran polyalcohol is obtained from a ratio occupied by the integrated intensity of signals at the C-1 position and the C-5 position, at which carboxymethyl group is bound to the side chain moiety, in the total integrated intensity of the signals at the C-1 position and the C-5 position.

As the spacer constituting the DDS compound of the present invention, a spacer containing one amino acid residue, or a spacer containing two to eight amino acid residues linked by peptide bond(s) can be used. The spacer has the form of a residue of one amino acid, which means a residue obtained by removing one hydrogen atom and one hydroxyl group from an amino group and a carboxyl group of the amino acid, respectively, or a residue of an oligopeptide containing two to eight amino acid residues linked by peptide bond(s), which means a residue obtained by removing one hydrogen atom and one hydroxyl group from the N-terminal amino group and the C-terminal carboxyl group, respectively. The spacer binds to the amino group at the 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione by a peptide bond at the C-terminal of the spacer (or the α-carboxyl group when the spacer contains one amino acid).

Preferred spacers are those containing residues of oligopeptides comprised of two to six amino acid residues. Types of amino acids constituting the spacer are not particularly limited. For example, L- or D-amino acids, preferably L- amino acids can be used. β-alanine, ε-aminocaproic acid, γ-aminobutyric acid or the like may also be used as well as α-amino acids. These amino acids other than α-amino acids are preferably located close to the drug carrier in the spacer.

Where a spacer containing an oligopeptide residue is used, the amino acid sequence thereof is not particularly limited. Preferably used spacers include, for example, a spacer being a residue of a dipeptide represented by -X-Z-, wherein X represents a residue of a hydrophobic amino acid and Z represents a residue of a hydrophilic amino acid; and -X-Z- means a residue which consists of a dipeptide that is formed by a peptide bond between a hydrophobic amino acid (X) and a hydrophilic amino acid (Z) at the N-terminal side and the C-terminal side, respectively, and whose one hydrogen atom and one hydroxyl group are removed from the amino group at the N-terminal and the carboxyl group at the C-terminal, respectively, and a spacer containing a residue of the dipeptide as a partial peptide sequence. As the hydrophobic amino acid, for example, phenylalanine, tyrosine, leucine and the like can be used, and as the hydrophilic amino acid, for example, glycine, alanine and the like can be used. The spacer may have a repeated sequence of the dipeptide residues (for example, -X-Z-X-Z-, -X-Z-X-Z-X-Z- and the like).

By using the spacer containing such dipeptide structure, the spacer can be hydrolyzed in tumorous sites or inflammatory sites, which are considered abundant in peptidases, to release the drug compound at a high concentration in the sites immediately. Accordingly, the partial structure formed by binding the spacer containing the above dipeptide and the drug compound to each other is a preferred partial structure of the DDS compound of the present invention.

Specific examples of oligopeptide residues that can be used as the spacer are shown in the following table. However, spacers used for the DDS compounds of the present invention are not limited to those mentioned below. It can be readily understood that an ordinary skilled artisan can appropriately choose the type of a spacer so as to achieve an optimum releasing rate of a drug compound. [The left ends of the peptide sequences are N-terminals, and C-terminals (or α-carboxyl groups when the spacer contains one amino acid) are bound to the amino group at the 1-position of (1S,9S)-1-amino-9-ethyl-5-fluoro-2,3-dihydro-9-hydroxy-4-methyl-1H,12H-benzo[de]-pyrano[3',4':6,7]indolizino[1,2-b]quinoline-10,13(9H,15H)-dione by a peptide bond. D-Phe represents D-phenylalanine residue and the other amino acids represent L-amino acids. The degrees of the releasing rate were judged from the degree of appearance of efficacy of the DDS compounds carrying doxorubicin against Walker 256 tumor-bearing rats, or from free doxorubicin concentrations at tumorous sites of Walker 256 tumor-bearing rats.] Among them, -Gly-Gly-Phe-Gly- is most preferably used as a spacer for the DDS compound of the present invention

### (a) Spacers having high releasing rate

-Leu-Gly-
-Tyr-Gly-
-Phe-Gly-
-Gly-Phe-Gly-
-Gly-Gly-Phe-Gly-
-Gly-Phe-Gly-Gly-
-Phe-Gly-Gly-Gly-
-Phe-Phe-Gly-Gly-
-Gly-Gly-Gly-Phe-Gly-

### (b) Spacers having relatively high releasing rate

-Gly-Gly-Phe-Phe-
-Gly-Gly-Gly-Gly-Gly-Gly-

### (c) Spacers having relatively low releasing rate

-Phe-Phe-
-Ala-Gly-
-Pro-Gly-
-Gly-Gly-Gly-Phe-

### (d) Spacers having low releasing rate

-Gly-
-D-Phe-Gly-
-Gly-Phe-
-Ser-Gly-
-Gly-Gly-
-Gly-Gly-Gly-
-Gly-Gly-Gly-Gly-

(1S,9S)-1-Amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione can be synthesized by the method described in Japanese Patent Unexamined Publication (KOKAI) No. (Hei) 5-59061/1993. The introduced amount of the residue of the aforementioned drug compound into the DDS compound of the present invention is from 3.2% to 8.4% by weight, preferably from 5.6% to 7.6% by weight based on the weight of the DDS compound. The introduced amount of the aforementioned drug compound can readily be determined by one of ordinary skill in the art, for example, by absorption analysis.

The DDS compound of the present invention can specifically exhibit desired antineoplastic activity at tumorous sites, and can be used as an antineoplastic agent with high safety. A medicament comprising the DDS compound of the present invention may generally be filled in vials and the like in the form of a lyophilized product and the like, and provided for clinical use as preparations for parenteral administration such as injections or drip infusions which are dissolved upon use. However, the form of the pharmaceutical preparations of the medicament of the present invention is not limited to the aforementioned forms. For the manufacture of the aforementioned pharmaceutical preparations, pharmaceutical additives available in the field of the art, for example, solubilizers, pH modifiers, stabilizers and the like can be used. A dose of the medicament of the present invention is not particularly limited. For example, about 1 to 500 mg, preferably about 10 to 100 mg per m² of body surface area per day may be administered once a day, and the administration may preferably repeated every 3 to 4 weeks.

Although the method for preparing the DDS compound of the present invention is not particularly limited, the DDS compound can suitably be prepared according to the aforementioned method of preparation provided by the present invention. The method of the present invention comprises any one of the aforementioned steps (A) to (D) or two or more steps in combination, and most preferably comprises all of the steps (A) to (D). As most preferred embodiment of the present invention, the method comprising all of the steps (A) to (D) will be explained below. However, the scope of the present invention is not limited to this preferred embodiment.

The preferred method of the present invention comprises the steps of:
(A) adding an aqueous solution containing sodium periodate to an aqueous solution containing dextran at a temperature of 4°C ± 2°C to oxidize the dextran, and then adding the resulting reaction mixture to an aqueous solution containing sodium borohydride at a temperature not higher than 15°C to obtain a dextran polyalcohol;
(B) reacting the dextran polyalcohol obtained in the aforementioned step (A) with sodium monochloroacetate to prepare a carboxymethyldextran polyalcohol, characterized in that the end of the reaction for carboxymethylation is determined by capillary electrophoresis;
(C) condensing the amino group at the 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]-quinoline-10,13(9H,15H)-dione with the α-carboxyl group of an amino acid whose α-amino group is protected with tert-butoxycarbonyl group, or with the C-terminal carboxyl group of an oligopeptide containing two to eight amino acids whose N-terminal is protected with tert-butoxycarbonyl group, characterized in that 1-ethyl-3-(dimethylaminopropyl)carbodiimide or a salt thereof is used as a condensing agent; and
(D) condensing with a carboxymethyldextran polyalcohol obtained in the step (B) a deprotected compound obtained by eliminating tert-butoxycarbonyl group from the condensate which is obtained in the aforementioned step (C), characterized in that 1-ethyl-3-(dimethylaminopropyl)carbodiimide or a salt thereof is used as a condensing agent.

The step (A) is a step for obtaining a dextran polyalcohol from a dextran. The type of the dextran as a starting material is not particularly limited, and the dextran may optionally contain α-D-1,6-linkages. For example, dextrans containing α-D-1,6-linkages at a rate of 85% or more, 90% or more, or 95% or more can be used. As the dextrans used as starting materials, preferred examples include those having molecular weight of about 500,000 such as Dextran T500 (Pharmacia). The degree of polyalcoholization of the resulting dextran polyalcohol is not particularly limited. Preferably, dextrans are treated under a condition which enables substantially complete polyalcoholization.

In the aforementioned oxidation using sodium periodate, the molecular weight of the dextran polyalcohol may sometimes be decreased due to a raised temperature during the reaction. The method of the present invention is characterized by adding an aqueous solution containing sodium periodate to an aqueous solution containing dextran at a temperature of 4°C ± 2°C to prevent the decrease of the molecular weight. The aqueous solution containing dextran may further contain, for example, a buffering agent. When the aqueous solution containing sodium periodate is added, the addition rate is desirably controlled so as not to cause a raise in the temperature of the reaction mixture. Appropriate stirring is desirably carried out to prevent a partial raise in the temperature. The reaction is completed in about a few days to about 20 days, normally in about 10 days. The concentration of dextran in the reaction mixture is, for example, about a few grams to about 100 grams per liter of the reaction mixture, preferably about 10 grams per liter.

After the completion of the reaction, the resulting reaction mixture is added with ethylene glycol and the like to consume excess peracid, if needed, and the pH of the reaction mixture is adjusted to near neutral condition, for example, about pH 6.5, if desired. Then the reaction mixture is added to an aqueous solution containing sodium borohydride at a temperature of 15°C or lower to perform reduction. Also in the reduction, the molecular weight of the dextran polyalcohol may sometimes be decreased due to a raise in the temperature during the reaction. To suppress the decrease in the molecular weight, the method of the present invention is characterized by adding the reaction mixture obtained in the aforementioned oxidation to an aqueous solution containing sodium borohydride at a temperature of 15°C or lower. The addition rate is desirably controlled so as not to cause a raise in the temperature of the reaction mixture. Appropriate stirring is desirably carried out to prevent a partial raise in the temperature. The reaction is generally completed in a few hours to a few days, preferably in about one day when the reaction mixture is kept at a temperature of ice cooling after the addition.

It is desirable to fractionate a dextran polyalcohol having a desired molecular weight from the resulting reaction mixture and use the same as a material for the following step (B). For example, fractions of low molecular weight and high molecular weight are desirably removed by using an ultrafiltration membrane, and if desired, some steps such as desalting and concentration may be added. The desalting and concentration can also be carried out by using an ultrafiltration membrane.

The step (B) is to prepare a carboxymethyldextran polyalcohol having the weight-average molecular weight of from 240,000 to 480,000 based on the pullulan standard by carrying out carboxymethylation of the dextran polyalcohol obtained in the aforementioned step (A). The carboxymethylation of the dextran polyalcohol can be carried out, for example, by reacting hydroxyl groups of the dextran polyalcohol with a halogenoacetic acid such as chloroacetic acid and bromoacetic acid, or a salt thereof, preferably sodium salt of monochloroacetic acid, to achieve partial carboxymethylation of the hydroxyl groups of the dextran polyalcohol. For example, the dextran polyalcohol is dissolved in an inert solvent which does not participate in the reaction (e.g., water, N,N-dimethylformamide, or dimethyl sulfoxide), and the resulting solution is added with a halogenoacetic acid or a salt thereof in the presence of a base (e.g., sodium hydroxide or potassium hydroxide), and then the mixture is subjected to the reaction for several minutes to several days at a temperature of ice-cooling to 100°C. The reaction can preferably be carried out at 20°C for several hours to about one day. After the reaction is completed, the fractions of low molecular weight and high molecular weight are desirably removed by using an ultrafiltration membrane, and if desired, some steps such as desalting and concentration using an ultrafiltration membrane may be applied.

Although the degree of carboxymethylation of the carboxymethyldextran polyalcohol can be controlled to some extent by a reaction temperature for the carboxymethylation or an amount of the halogenoacetic acid or a salt thereof used as a reagent, the method of the present invention is characterized in that the end of the carboxymethylation is determined by capillary electrophoresis to more precisely regulate the degree of carboxymethylation so as to be in a range of from 0.14 to 0.47, or from 0.23 to 0.47.

The capillary electrophoresis (CE) is a method of carrying out electrophoresis generally in a capillary having an inside diameter of 100 µm or less made of fused silica (see, e.g., Yoshinobu Baba, "Bunseki" (Analysis), 342, 1995 and the like). For the capillary electrophoresis, several separation modes have been proposed such as capillary zone electrophoresis (CZE), electrokinetic chromatography (EKC), and capillary gel electrophoresis (CGE). Any of these separation modes may be used in the method of the present invention. Preferably, capillary zone electrophoresis can be use. Separation can be carried out after the inside of the capillary is filled with a buffering solution such as those containing phosphoric acid, citric acid, boric acid and the like. According to this method, the charge per unit molecular weight can be accurately determined, and the degree of carboxymethylation of a sample from the aforementioned reaction mixture can be determined in a short period of time and in a high sensitivity. The details of the method will be specifically described in the examples of the specification. Accordingly, those skilled in the art can readily and accurately determine the end of the reaction for carboxymethylation (the degree of carboxymethylation being from 0.14 to 0.47 or from 0.23 to 0.47) by referring to the general explanations of the aforementioned publications and other publications, and by according to the specific methods described in the examples of the specification, and if necessary, by appropriately modifying or altering said methods.

As already explained, when the carboxymethylation degree of the carboxymethyldextran polyalcohol is measured by the capillary electrophoresis, a calibration curve obtained by using a standard substance can be employed. The calibration curve can be obtained by either the decomposition method or the NMR method. The decomposition method and the NMR method may sometimes give different measured values of the degree of carboxymethylation for the same standard substance. In general, a value of the degree of carboxymethylation determined by the NMR method tends to be lower by approximately 0.09 compared to a value determined by the decomposition method. Accordingly, when a calibration curve obtained by the NMR method is used, the degree of carboxymethylation is desirably in a range of from 0.14 to 0.38.

The step (C) is to condense the amino group at the 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione with the C-terminal carboxyl group of the oligopeptide (or the α-carboxyl group when an amino acid is used) used as a spacer. The aforementioned oligopeptide or the amino acid used as a spacer is required to be protected by tert-butoxycarbonyl group at the N-terminal amino group or the α-amino group, respectively. Means for the protection are well-known in the art and commonly used.

For carrying out the aforementioned condensation, the method of the present invention is characterized to use 1-ethyl-3-(dimethylaminopropyl)carbodiimide (EPCI) or a salt thereof, preferably 1-ethyl-3-(dimethylaminopropyl)carbodiimide hydrochloride, as a condensing agent. When the aforementioned condensing agent is used, reaction operations can be simplified and a reaction time can be much shortened compared to a process wherein a N,N'-dicycloalkylcarbodiimide such as N,N'-dicyclohexylcarbodiimide (DCC) is used as a condensing agent. More specifically, centrifugation and column operations for removing the condensing agent can be avoided, and a reaction time can be shortened to about 1/5 as compared to a process wherein DCC is used. A substrate concentration can also be increased about 5 times as compared to the process wherein DCC is used. In particular, a cost can be much reduced in a large scale synthesis for industrial application by decreasing an amount of the reagent, shortening reaction time and the like.

The aforementioned reaction can be carried out in the same manner as condensation for formation of a peptide bond using a common condensing agent except that EPCI or a salt thereof is used as the condensing agent. The reaction can be carried out by using 1 to 1.5 equivalents of a tert-butoxycarbonylated amino acid or a tert-butoxycarbonylated oligopeptide based on the aforementioned drug compound in an inert solvent such as dimethylformamide. The reaction is generally completed in a few hours to about one day at room temperature, preferably in about 3 hours at room temperature. A concentration of the drug compound in a reaction mixture is not particularly limited. Generally, the concentration is about 50 to 200 g per liter, preferably about 100 to 150 g per liter.

The step (D) is to condense a deprotected compound, which is obtained by eliminating tert-butoxycarbonyl group from the condensate obtained in the aforementioned step (C), with the carboxymethyldextran polyalcohol obtained in the step (B). The method of removing tert-butoxycarbonyl group is well-known to one of ordinary skill in the art and commonly used. For example, a method comprising treatment with trifluoroacetic acid is preferred. When the deprotected compound is purified, for example, washing can be carried out by using isopropyl ether and the like.

The method of the present invention is characterized in that 1-ethyl-3-(dimethylaminopropyl)carbodiimide (EPCI) or a salt thereof, preferably 1-ethyl-3-(dimethylaminopropyl)carbodiimide hydrochloride, is used as a condensing agent when the N-terminal amino group (or the α-amino acid when one amino acid is used as a spacer) of the spacer bound to the drug compound is condensed with the carboxyl group of the carboxymethyldextran polyalcohol. When the aforementioned condensing agent is used, centrifugation and column operations for removing the condensing agent can be avoided, and a reaction time can be much shortened compared to a process wherein a N,N'-dicycloalkylcarbodiimide such as N,N'-dicyclohexylcarbodiimide (DCC) is used as a condensing agent. Therefore, a cost can be much reduced in a large scale synthesis for industrial application. The end of the reaction may be determined by HPLC.

The aforementioned reaction can be carried out in the same manner as a condensation for formation of a peptide bond using a common condensing agent except that EPCI or a salt thereof is used as the condensing agent. The reaction can be carried out by using about 0.1 to 0.2 part by weight of the amino acid or the oligopeptide bound to the drug compound relative to 1 part by weight of the carboxymethyldextran polyalcohol in an inert solvent such as water-containing methanol. The reaction is generally completed in a few hours to about one day at room temperature, preferably in about two to three hours at room temperature.

Specific examples of the method of the present invention are described in the examples of the specification, and accordingly, those skilled in the art can carry out the method of the present invention by referring to the above general explanations and the specific explanations in the examples, and if necessary, by adding modifications or alterations to the disclosed methods. In addition, it should be understood that a reaction temperature, a reaction time, concentrations of reagents and the like can be appropriately chosen by those skilled in the art within the scope of the present invention.

A medicament comprising the DDS compound of the present invention may generally be filled in vials and the like in the form of a lyophilized product and the like, and provided for clinical use as a preparation for parenteral administration such as an injection or a drip infusion which is dissolved upon use as a medicament for therapeutic treatment of tumors. As for the use of the DDS compound of the present invention for therapeutic treatment of tumors, the disclosure of International Publication WO97/46260 is incorporated by reference in the disclosure of the present specification. However, the forms of pharmaceutical preparations of the medicament of the present invention are not limited to the aforementioned forms. For the manufacture of the aforementioned pharmaceutical preparations, pharmaceutical additives available in the field of the art, for example, solubilizers, pH modifiers, stabilizers and the like, can be used. Although the dose of the above medicament is not particularly limited, about 0.1 to 100 mg, preferably about 1 to 30 mg per m² of body surface area per day may parenterally be administered once a day, and the administration may preferably be repeated every 3 to 4 weeks.

### Examples

The present invention will be explained more specifically by examples. However, the scope of the present invention is not limited to the following examples. In the examples, (1S,9S)-1-amino-9-ethyl-5-fluoro-2,3-dihydro-9-hydroxy-4-methyl-1H, 12H-benzo[de]pyrano[3',4':6,7]indolizino[1,2-b]quinoline-10,13(9H,15H)-dione is sometimes referred to as "the drug compound A", and "Gly-Gly-Phe-Gly" means glycyl-glycyl-phenylalanyl-glycine or its residue. The DDS compound used in the test examples was a DDS compound in which the aforementioned drug compound and a carboxymethyldextran polyalcohol are bound to each other by means the tetrapeptide spacer (Gly-Gly-Phe-Gly) and prepared so as to have a macromolecular carrier with a different carboxymethylation degree and a different molecular weight.

### Example 1: Synthesis of dextran polyalcohol (Dex-PA)

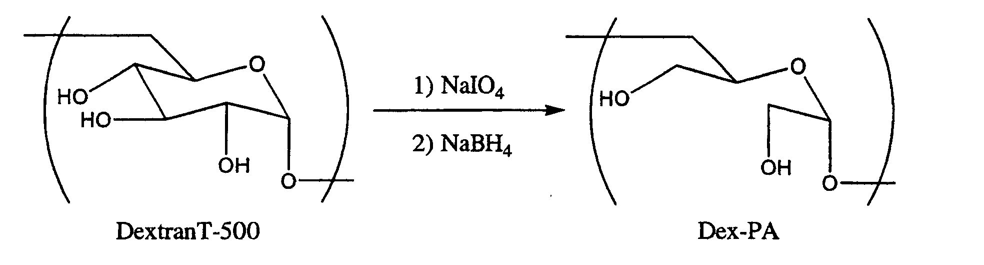

Dextran-T500 (Pharmacia, 300 g) was dissolved in 0.2 M acetic buffer (15 l) adjusted to pH 5.5, and NaIO₄ (990 g) was dissolved in pure water (15 l), and the resulting solutions were allowed to stand in a low-temperature room (about 4°C) overnight. On the next day, the NaIO₄ solution (3.5°C) was gradually poured to the solution of Dextran-T500 (3.5°C) so as not to cause a raise in the temperature (not more than 7.0°C), and after the addition, the mixture was stirred (100 rpm) without further treatment in the low-temperature room. After the mixture was stirred for 10 days, ethylene glycol (210 ml) was added to the mixture and stirring was continued for 2 hours. Disappearance of the peracid was verified by using a Peroxid test paper, and then the reaction mixture was adjusted to pH 6.5 with 10% NaOH. Then, the reaction mixture was added dropwise to a NaBH₄ solution (420 g, 12 l) with ice cooling. During the addition, a temperature in the system was kept under 15°C and the dropwise addition was carried out over 3 hours. Then, the reaction mixture was stirred in the low-temperature room overnight, and on the next day, the mixture was adjusted to pH 5.5 with acetic acid. Stirring was continued for additional 1 hour, and the pH of the mixture was adjusted to 7.0 with 10% NaOH. The resulting reaction mixture was treated with an ultrafiltration membrane by Paul Filtron (1000 k) to remove fine particles and macromolecular fractions. The mixture was further treated with a Millipore ultrafiltration membrane (50 k) (using about 90 to 100 1 of pure water) for desalting and concentration, and the mixture was concentrated to 1,997 ml under being monitored by HPLC. A part (1 ml ×3) of the mixture was sampled and lyophilized to give 60.1 mg of the product, and accordingly, a total yield was calculated as 120 g.

### Example 2: Synthesis of carboxymethyldextran polyalcohol (CM-Dex-PA)

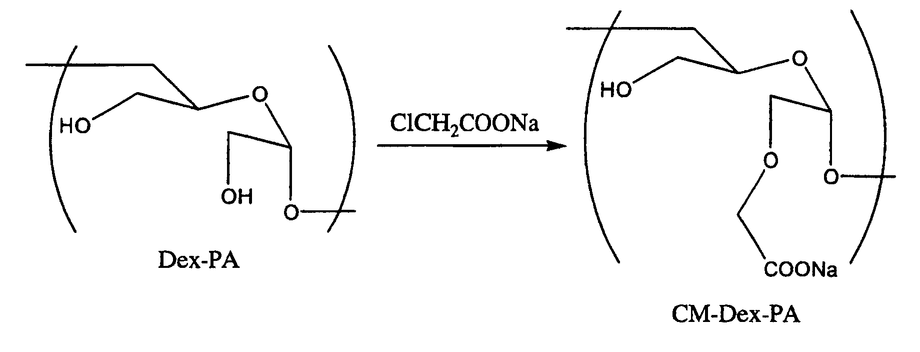

### (A) Synthesis of carboxymethyldextran polyalcohol (CM-Dex-PA)

NaOH (193 g) was dissolved in pure water (1,537 ml), and the solution was added with an aqueous solution of a dextran polyalcohol while the temperature of the solution was maintained at 25°C. Stirring was continued while the temperature of the system was kept at 25°C. The mixture was gradually added with sodium monochloroacetate, and the mixture was stirred at the same temperature for 15 hours. The end of the reaction was verified by the capillary electrophoresis, and then the mixture was adjusted to about pH 8.0 with acetic acid and subjected to ultrafiltration. Macromolecules were first removed by using a membrane of 1,000 k, and then low-molecular compounds (reagents and salts) were removed by using a membrane of 50 k and the mixture was concentrated. In these operations, progress of the removal of the low-molecular compounds was monitored occasionally by HPLC. After the removal of most of the low-molecular compounds was verified, the ultrafiltration was finished. The carboxymethyldextran polyalcohol solution was concentrated to 3,770 ml. 1 ml of the concentrated solution was lyophilized to give 32.1 mg of the product, and accordingly, a total yield was calculated as 121 g.

### (B) Measurement of the carboxymethylation degree by the capillary electrophoresis

### -Method 1

For the capillary electrophoresis, a photodiode array detector of 190 nm-300 nm (195 nm detected) and a capillary made of fused silica having inside diameter of 75 µm, effective length of 500 mm and total length of 670 mm were used. 20 mM aqueous sodium tetraborate was used as an electrophoresis solution. Samples were prepared at concentration of 2 mg/ml using 0.02% aqueous sodium azide. As samples, three lots prepared by applying reaction time of 19 hours, 19.5 hours, and 20 hours were used. Calibration curves were prepared by using three different carboxymethyldextran polyalcohols as standard substances whose respective carboxymethylation degrees were found to be 0.22, 0.42, and 0.62 by the decomposition method. The standard substances had retention time (minutes) of 4.496, 5.442, and 6.600. The samples were found to have retention time (minutes) of 5.325, 5.400, and 5.446, respectively. From these results, the degree of carboxymethylation of each sample was determined as 0.38, 0.40, and 0.41.

### Example 3: Synthesis of tert-butoxycarbonyl(Boc)-Gly-Gly-Phe-Gly-drug compound A

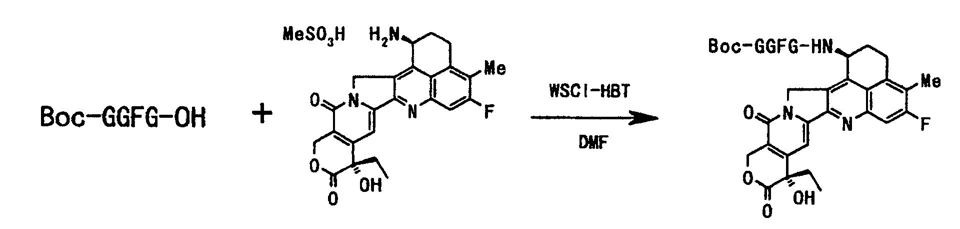

Methanesulfonate of Drug compound A (80 g) and tert-butoxycarbonyl-Gly-Gly-Phe-Gly-OH (68 g) were suspended in N,N-dimethylformamide (1,200 ml). The suspension was added with triethylamine (48 ml), 1-ethyl-3-(dimethylaminopropyl)-carbodiimide hydrochloride (EPCI·HCL, 29.6 g), and hydroxybenzotriazole (HBT, 20.8 g) with stirring under ice cooling, and then stirring was continued at room temperature. The end of the reaction was verified by HPLC, and then the reaction mixture was added dropwise with water (800 ml) over 30 minutes under ice cooling with stirring so as to maintain an internal temperature at 20°C or lower to allow deposition of crystals. Then, water (1,200 ml) was further added dropwise to the mixture. The solution was adjusted to pH 7 with acetic acid. The deposited solids were collected by filtration and washed with water, and the resulting crystals were dried under reduced pressure to obtain the title compound (120.4 g, quantitatively).
¹H-NMR (DMSO-d₆/TMS) δ (ppm): 0.97 (3H, m), 1.11 (2H, d, J=6.3Hz), 1.41 (9H, s), 1.91 (2H, m), 2.05 (1H, m), 2.33 (4H, m), 2.95-3.10 (4H, m), 3.58-3.72 (2H, m), 3.8 (1H, m), 4.03 (1H, m), 4.34 (1H, m), 4.74 (1H, m), 5.13 (1H, m), 5.33 (1H, m), 5.58 (2H, m), 7.18 (5H, m), 7.49 (2H, m).

### Example 4: Synthesis of H-Gly-Gly-Phe-Gly-drug compound A·trifluoroacetate

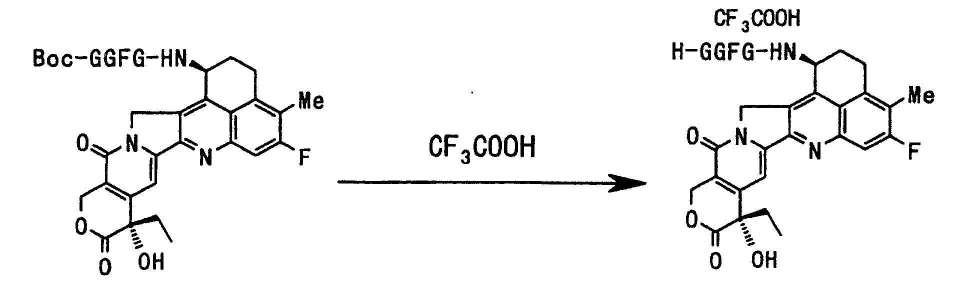

The tert-butoxycarbonyl-Gly-Gly-Phe-Gly-drug compound A obtained in Example 3 above (120 g) was added dropwise with trifluoroacetic acid (360 ml) under ice cooling. After the tert-butoxycarbonyl-Gly-Gly-Phe-Gly-drug compound A was completely dissolved, the end of des-tert-butoxycarbonylation was verified by HPLC. The reaction mixture was added dropwise with methanol (360 ml) and isopropyl ether (720 ml) so as to keep an internal temperature between 0°C and 15°C. The deposited crystals were collected by filtration and washed three times with ethyl acetate (500 ml). The resulting crystals were dissolved in methanol containing 20% water (400 ml) at an internal temperature of 50°C or lower, and then the solution was added with ethyl acetate (400 ml) and isopropyl ether (800 ml) to allow recrystallization. The crystals were collected by filtration and dissolved in water-containing methanol (400 ml), and then added with activated charcoal (4.4 g) for decolorization. The solution was filtered, and the filtrate was added with ethyl acetate (400 ml) and then with isopropyl ether (800 ml) at 55°C or less for recrystallization. The crystals obtained by filtration was dried under reduced pressure to obtain H-Gly-Gly-Phe-Gly-drug compound A (111.8 g, 90% based on the drug compound A).
¹H-NMR (DMSO-d₆/TMS) δ (ppm): 0.87 (3H, m), 1.87 (2H, m), 2.17 (2H, m), 2.37 (3H, m), 2.74 (1H, m), 3.00 (1H, m), 3.16 (1H, m), 3.58 (2H, s), 3.65-3.91 (4H, m), 4.48 (1H, m), 5.2 (2H, s), 5.39 (2H, m), 5.58 (1H, m), 6.53 (1H, s), 7.21 (5H, m), 7.75 (1H, d, J=10.9Hz), 8.06 (2H, s), 8.28 (1H, d, J=8.2Hz), 8.49 (1H, m), 8.52 (1H, m).

### Example 5: Synthesis of the DDS compound of the present invention

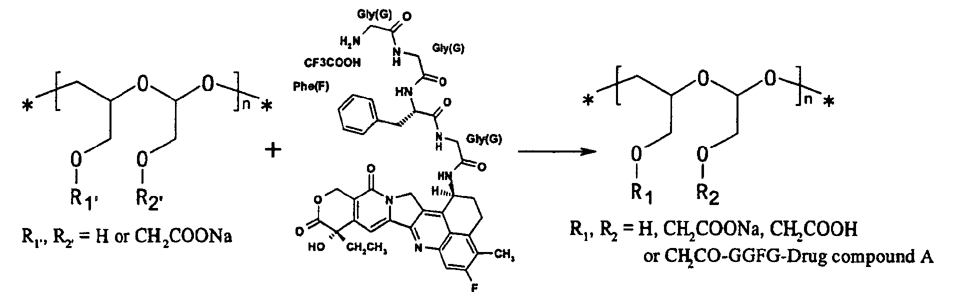

An aqueous solution containing a carboxymethyldextran polyalcohol (800 g) was added with pure water to give 32 L of a solution in total including the aqueous solution of carboxymethyldextran polyalcohol, and the solution was further added with methanol (60 L). The mixture was added with 20% water-containing methanol (4 l) in which the H-Gly-Gly-Phe-Gly-drug compound A obtained in Example 4 (133 g) and hydroxybenzotriazole (23.4 g) were dissolved. The mixture was added with 1-ethyl-3-(dimethylaminopropyl)carbodiimide hydrochloride (33.1 g) and then adjusted to pH 6.8 to 7.2 with 1 N NaOH. This mixture was allowed to react at room temperature (23°C±5°C) for 2 to 3 hours. 1-Ethyl-3-(dimethylaminopropyl)carbodiimide hydrochloride (8.1 g) was further added, and the mixture was adjusted to pH 6.8 to 7.2 with 1 N HCl, and subsequently allowed to react for about 2 to 3 hours. Moreover, 1-ethyl-3-(dimethylaminopropyl)carbodiimide hydrochloride (5.6 g) was added, and the mixture was adjusted to pH 6.8 to 7.2 with 1 N HCl, and subsequently allowed to react for about 1 hour. After the reaction was completed, the reaction mixture was adjusted to pH 8.7 to 9.2 with 1 N NaOH and stored at 10°C or less. Then, the solution was desalted and concentrated by using an ultrafiltration membrane (50 k) and followed by microfiltration using a membrane filter. The resulting solution was lyophilized to obtain the DDS compound of the present invention (880 g).

### Example 6: Effect of reaction temperature on a decrease in the molecular weight of dextran polyalcohol (oxidation)

Oxidation of dextran was carried out according to Example 1 in a scale of the substrate concentration of 1% and 20 g. Reaction temperature was set at 4, 8, 12 or 15°C and reaction time was varied. Retention time of each product was measured by gel filtration chromatography. As shown in Table 1, apparent delay in the retention time caused by a decrease in molecular weight was recognized on the 6th day when the reaction temperature was 12°C and 15°C. Also at 8°C, apparent delay in the retention time was recognized on the 10th day.

**Table 1**

| Reaction temperature/time | 3 days | 6 days | 10 days |
|---|---|---|---|
| 4°C | 10.46 minutes | 10.44 minutes | 10.53 minutes |
| 8°C | 10.52 | 10.63 | 10.83 |
| 12°C | 10.45 | 10.63 | ----- |
| 15°C | 10.59 | 10.92 | ----- |

### Example 7: Effect of reaction temperature on a decrease in the molecular weight of dextran polyalcohol (oxidation)

In view of the results of Example 6, the same experiment was carried out with setting the temperature range more finely (4°C, 1°C, 6.5°C). As shown in Table 2, the reaction proceeded at all the temperatures without occurrence of a decrease in the molecular weight. However, deposition of salts in the reaction system increased when the reaction was carried out at 1°C. From the results above, a safe temperature range for the reaction was considered to be from 2 to 6°C.

**Table 2**

| Reaction temperature/time | 3 days | 6 days | 10 days |
|---|---|---|---|
| 4°C | 10.40 minutes | 10.39 minutes | 10.49 minutes |
| 1°C | 10.42 | 10.39 | 10.45 |
| 6.5°C | 10.40 | 10.36 | 10.39 |

### Example 8: Effect of reaction temperature on a decrease in the molecular weight of dextran polyalcohol (reduction)

According to Example 1, the reduction after the oxidation of dextran was carried out. The reaction was carried out by setting reaction temperature at 10, 15, 20 or 30°C for from 12 to 24 hours, and then retention time of each product was measured by gel filtration chromatography. As shown in Table 3, an apparent decrease in the molecular weight was observed at the reaction temperatures over 15°C.

**Table 3**

| Reaction temperature | Judgment by gel filtration chromatography |
|---|---|
| 10°C | No decrease in molecular weight |
| 15°C | Slight decrease in molecular weight |
| 20°C | Decrease in molecular weight |
| 30°C | Significant decrease in molecular weight |

### Example 9: Measured value fluctuation of the degree of carboxymethylation of the DDS compound depending on the method of measurement of the degree of carboxymethylation

Calibration curves were prepared by the decomposition method and the NMR method using three different carboxymethyldextran polyalcohols as standard substances. The carboxymethylation degree of each of the DDS compounds (three lots) was measured in the same manner as in Example 2(B) using the calibration curves. The results are shown below. When the calibration curves obtained by the NMR method were used, measured values of the carboxymethylation degree decreased by 0.09 compared to that obtained by the decomposition method for all of the lots.

**Table 4**

| Degrees of carboxymethylation of the standard substances (carboxymethyldextran polyalcohol) | | | |
|---|---|---|---|
| | Standard No.1 | Standard No.2 | Standard No.3 |
| Decomposition method | 0.333 | 0.439 | 0.584 |
| NMR method | 0.248 | 0.360 | 0.523 |

**Table 5**

| Degrees of carboxymethylation of the DDS compounds | | | |
|---|---|---|---|
| | Lot 1 | Lot 2 | Lot 3 |
| Decomposition method | 0.36 | 0.37 | 0.37 |
| NMR method | 0.27 | 0.28 | 0.28 |
| Difference | Δ 0.09 | Δ 0.09 | Δ 0.09 |

Test examples will be shown below. The degrees of carboxymethylation shown in the test examples are those obtained by the decomposition method.

### Test Example 1

### (A) Method

As animals, male BALB/c mice of 6 weeks old (Nippon SLC Co.) were fed on a commercially available feed and water ad libitum, conditioned for one week, and then subjected to the test. As tumor cells, mouse tumor cells of Meth A fibrosarcoma were subcultured intraperitoneally using BALB/c mice as syngeneic mice every one week. The tumor cells were collected from the mouse abdominal cavity using the Hanks' medium with an endotoxin concentration of 50 pg/ml or less (HBSS, Gibco-BRL). The cells were washed several times by centrifugation (about 600 rpm, 5 to 10 minutes, 4°C), then suspended in the HBSS medium and transplanted to the mouse intraperitoneally in a ratio of 1×10⁶ cells/0.1 ml per mouse.

For antineoplastic test, the Meth A cells were subcutaneously transplanted to the right inguinal region of mice in a ratio of 1×10⁶ cells/0.1 ml per mouse (day 0). The mice were divided into groups each consisting of 6 or 7 mice so that a group average of estimated tumor weights (ETW = L×W²/2 mg), calculated from the length (L) and width (W) of tumors measured using a caliper on the 7th day or 12th day after the transplantation, was about 100 mg. Test samples were intravenously administered as single administration or as four-times administration every 4 days. On the 21st day or 26th day after the tumor transplantation, the mice were sacrificed by cervical dislocation, and each tumor was isolated and weighed. The inhibitory effect on tumor proliferation reproduction (IR) was calculated from the value of tumor weight using the formula: IR = (1-TWt/TWc) ×100 (%) (TWt represents a mean tumor weight of the group administered with the test sample; and TWc represents that of the control group). When IR was not less than 58%, the test sample was judged to be effective in antineoplastic activity. The significance test between the control group and the group administered with the test substance was carried out by the Dunnet method.

Furthermore, in order to evaluate a potency of side effects of the test sample, a body weight loss (BWL) and the ratio of the number of mice died from toxicity to that of mice used (D/U) were used as toxic parameters. For BWL , the formula: BWL = (1 - BWn/BWs) ×100 (%) was used, and the value was calculated from a mean body weight of the mice at the start of the administration (BWs) and that of the mice on the day "n". The maximum of BWL was defined as BWLₘₐₓ. When body weight loss was not observed as compared to the mice at the day of starting the administration, BWLₘₐₓ was shown as 0 or less (<0). The test sample was dissolved in physiological saline for injection according to the Japanese Pharmacopoeia and intravenously administered at the administration volume of 10 or 20 ml/kg.

### (B) Results

When the single administration test was carried out for the DDS compound in which the molecular weight of the carboxymethyldextran polyalcohol was in the range of 48,000 to 457,000 (the degree of carboxymethylation: 0.37 to 0.46; the introduced amount of the drug: 4.6 to 6.4% by weight), a significant antineoplastic effect of 58% or more was recognized stably at low doses when the molecular weight of the carboxymethyldextran polyalcohol was from 200,000 to 300,000. The minimum effective amount was increased when the molecular weight was less than 200,000. When the molecular weight was less than 50,000, the toxicity and the antineoplastic effect were reduced, which was presumably due to urinary excretion of the text sample. Accordingly, it was concluded that a stable antineoplastic effect can be obtained even at a low dose if the molecular weight of the carboxymethyldextran polyalcohol is 200,000 or more. On the other hand, when the molecular weight of the carboxymethyldextran polyalcohol was beyond 500,000, problems arose such as low stability against physical damage because of its viscosity. From these results, it was concluded that the molecular weight of the carboxymethyldextran polyalcohol in the DDS compound is required to be in the range of from 50,000 to 500,000, and that the weight-average molecular weight of the carboxymethyldextran polyalcohol based on the pullulan standard is desirably in the range of from 240,000 to 480,000 to achieve the desired antineoplastic effect and produce stable products.

### Test Example 2

69 Male BALB/c mice of 7 weeks old (Nippon SLC Co.) were conditioned for one week, and each group consisting of 5 mice was administered with the DDS compound having different degree of carboxymethylation (the introduced amount of the drug: 5.3 to 6.3% by weight; the molecular weight: 270,000 to 330,000). The average body weight at the administration was from 21.1 g to 25.4 g. Each five mice were placed in a cage made of aluminum in a room set at the room temperature of 23 ± 2°C, the humidity of 55 ± 20%, and the lighting period of 12 hours (from 8:00 to 20:00), and bred by feeding ad libitum commercially available solid feed (F2, Funabashi Farm) and tap water containing chlorine. The DDS compounds were dissolved in physiological saline according to the Japanese Pharmacopoeia and administered to the caudal vein in the liquid volume of 1 ml/kg at a concentration of from 1.02 to 1.36 mg/ml.

Symptoms of the animals were observed once a day for 15 days including the day of administration, and the body weight was measured before the administration and on the 3rd, 7th, 10th and 14th day after the administration. Dead mice were immediately subjected to autopsy, and survival mice were sacrificed by cutting the abdominal aorta under ether anesthetization to allow bleeding to death. Organs in the whole body of the mice were macroscopically observed. As to the data of body weight, an average of the group ± a standard deviation was calculated, and then the statistical analysis was carried out at significance level of 5%. As a result, the maximum tolerant doses (MTD) of the DDS compounds having the degree of carboxymethylation of 0.38, 0.43 and 0.47 were 11.7, 11.7 and 10.3 mg/kg, respectively, which suggests that the toxicity tends to be increased when the degree of carboxymethylation exceeds 0.43. In addition, the toxicity was similarly evaluated by using the DDS compound having the degree of carboxymethylation of 0.53. As a result, in the 10 mg/kg administered group, 3 mice in 6 died, and the weight loss and the fatal toxicity were remarkably enhanced. Whilst, MTD of the DDS compound having the degree of carboxymethylation of 0.23 was equivalent to that of the DDS compound having the degree of carboxymethylation of 0.38. Accordingly, it was concluded that the degree of carboxymethylation of the DDS compound is preferably in the range of from 0.23 to 0.47 from a viewpoint of safety.

### Test Example 3

The antineoplastic test was carried out in the same manner as in the aforementioned test example 2 for the DDS compounds in which the introduced amount of the residue of the drug compound was from 3.2 to 15% by weight (the degree of carboxymethylation: 0.37 to 0.40, the molecular weight: 260,000 to 320,000). As a result, in the 1.25 mg/kg administered group, IR was 80% or more when the introduced ratio of 3.2 to 7.3% by weight, which indicates effectiveness of the compound, whereas, it was observed that the effectiveness tended to be apparently decreased compared to the above test when the introduced ratio exceeded 8.4% by weight (up to 15%). When the introduced ratio exceeded 8.4% by weight, almost the same effectiveness was observed in the 2.5 or 5 mg/kg administered group as that of the other complexes; whereas enhancement of the fatal toxicity was observed in the 10 mg/kg administered group. From these results, it was concluded that the introduced amount of the residue of the drug compound is desirably in a range of from 3.2 to 8.4% by weight.

### Industrial Availability

The DDS compound of the present invention has high safety and a broad effective range and is extremely useful as an antineoplastic agent for clinical use. In addition, according to the method of the present invention, the aforementioned DDS compound with high quality can efficiently be prepared in a high yield, which is suitable for industrial application.

## Claims

1. A DDS compound in which amino group at 1-position of (1S,9S)-1-amino-9-ethyl-5-fluoro-2,3-dihydro-9-hydroxy-4-methyl-1H,12H-benzo[de]pyrano[3',4':6,7]-indolizino[1,2-b]quinoline-10,13(9H,15H)-dione is bound to a carboxyl group of a carboxymethyldextran polyalcohol with a spacer containing one amino acid or two to eight amino acids linked by peptide bond(s), **characterized in that**
(1) an introduced amount of residue of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione is in a range of from 3.2% to 8.4% by weight of total weight of the DDS compound;
(2) a weight-average molecular weight of the carboxymethyldextran polyalcohol based on pullulan standard is in a range of from 240,000 to 480,000; and
(3) degree of carboxymethylation of the carboxymethyldextran polyalcohol is in a range of from 0.23 to 0.47.

2. A DDS compound in which amino group at 1-position of (1S,9S)-1-amino-9-ethyl-5-fluoro-2,3-dihydro-9-hydroxy-4-methyl-1H,12H-benzo[de]pyrano[3',4':6,7]-indolizino[1,2-b]quinoline-10,13(9H,15H)-dione is bound to a carboxyl group of a carboxymethyldextran polyalcohol with a spacer containing one amino acid or two to eight amino acids linked by peptide bond(s), **characterized in that**
(1) an introduced amount of residue of (1S,9S)-1-amino-9-ethyl-5-fluoro-2,3-dihydro-9-hydroxy-4-methyl-lH,12H-benzo[de]pyrano[3',4':6,7]indolizino[1,2-b]quinoline-10,13(9H,15H)-dione is in a range from 3.2% to 8.4% by weight of total weight of the DDS compound;
(2) a weight-average molecular weight of the carboxymethyldextran polyalcohol based on pullulan standard is in a range of from 240,000 to 480,000; and
(3) degree of carboxymethylation of the carboxymethyldextran polyalcohol is in a range of from 0.14 to 0.47.

3. A DDS compound in which amino group at 1-position of (1S,9S)-1-amino-9-ethyl-5-fluoro-2,3-dihydro-9-hydroxy-4-methyl-1H,12H-benzo[de]pyrano[3',4':6,7]-indolizino[1,2-b]quinoline-10,13(9H,15H)-dione is bound to a carboxyl group of a carboxymethyldextran polyalcohol with a spacer containing one amino acid or two to eight amino acids linked by peptide bond(s), **characterized in that**
(1) an introduced amount of residue of (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione is in a range of from 3.2% to 8.4% by weight of total weight of the DDS compound;
(2) a weight-average molecular weight of the carboxymethyldextran polyalcohol based on pullulan standard is in a range of from 240,000 to 480,000; and
(3) degree of carboxymethylation of the carboxymethyldextran polyalcohol is in a range of from 0.14 to 0.38.

4. The DDS compound according to any one of claims 1 to 3, wherein the degree of carboxymethylation in the above (3) is measured by capillary electrophoresis using a calibration curve which is obtained by measuring a carboxymethyldextran polyalcohol as a standard substance by a decomposition method or an NMR method.

5. The DDS compound according to claim 1, wherein the degree of carboxymethylation in the above (3) is measured by the capillary electrophoresis using a calibration curve which is obtained by measuring a carboxymethyldextran polyalcohol as a standard substance by the decomposition method.

6. The DDS compound according to claim 3, wherein the degree of carboxymethylation in the above (3) is measured by the capillary electrophoresis using a calibration curve which is obtained by measuring a carboxymethyldextran polyalcohol as a standard substance by the NMR method.

7. An antineoplastic agent which comprises the DDS compound according to any one of claims 1 to 6.

8. A method for preparing the DDS compound according to any one of claims 1 to 6, which comprises one or more steps selected from the group consisting of the following steps of:
(A) adding an aqueous solution containing sodium periodate to an aqueous solution containing dextran at a temperature of 4°C ± 2°C to oxidize the dextran, and then adding the resulting reaction mixture to an aqueous solution containing sodium borohydride at a temperature not higher than 15°C to obtain a dextran polyalcohol;
(B) reacting a dextran polyalcohol with sodium monochloroacetate to prepare a carboxymethyldextran polyalcohol, **characterized in that** an end of carboxymethylation is determined by capillary electrophoresis;
(C) condensing amino group at 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]-quinoline-10,13(9H,15H)-dione with α-carboxyl group of an amino acid whose α-amino group is protected with tert-butoxycarbonyl group, or C-terminal carboxyl group of an oligopeptide containing two to eight amino acids whose N-terminal is protected with tert-butoxycarbonyl group, **characterized in that** 1-ethyl-3-(dimethylaminopropyl)carbodiimide or a salt thereof is used as a condensing agent; and
(D) condensing with a carboxymethyldextran polyalcohol a deprotected compound obtained by eliminating tert-butoxycarbonyl group from a condensate in which the amino group at the 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]-quinoline-10,13(9H,15H)-dione is condensed with α-carboxyl group of an amino acid whose α-amino group is protected with tert-butoxycarbonyl group, or C-terminal carboxyl group of an oligopeptide containing two to eight amino acids whose N-terminal is protected with tert-butoxycarbonyl group, **characterized in that** 1-ethyl-3-(dimethylaminopropyl)carbodiimide or a salt thereof is used as a condensing agent.

9. A method for preparing the DDS compound according to any one of claims 1 to 6, which comprises the following steps of:
(A) adding an aqueous solution containing sodium periodate to an aqueous solution containing dextran at a temperature of 4°C ± 2°C to oxidize the dextran, and then adding the resulting reaction mixture to an aqueous solution containing sodium borohydride at a temperature not higher than 15°C to obtain a dextran polyalcohol;
(B) reacting the dextran polyalcohol obtained in the step (A) with sodium monochloroacetate to prepare a carboxymethyldextran polyalcohol, **characterized in that** an end of carboxymethylation is determined by capillary electrophoresis;
(C) condensing amino group at 1-position of (1S,9S)-1-amino-9-ethyl-5-fluoro-2,3-dihydro-9-hydroxy-4-methyl-1H,12H-benzo[de]pyrano[3',4':6,7]indolizino[1,2-b]-quinoline-10,13(9H,15H)-dione with α-carboxyl group of an amino acid whose α-amino group is protected with tert-butoxycarbonyl group, or C-terminal carboxyl group of an oligopeptide containing two to eight amino acids whose N-terminal is protected with tert-butoxycarbonyl group, **characterized in that** 1-ethyl-3-(dimethylaminopropyl)carbodiimide or a salt thereof is used as a condensing agent; and
(D) condensing with a carboxymethyldextran polyalcohol a deprotected compound obtained by eliminating tert-butoxycarbonyl group from the condensate which is obtained in the step (C), **characterized in that** 1-ethyl-3-(dimethylaminopropyl)carbodiimide or a salt thereof is used as a condensing agent.

10. A carboxymethyldextran polyalcohol which is used for preparation of the DDS compound according to any one of claims 1 to 6, wherein a weight-average molecular weight based on pullulan standard is in a range of from 240,000 to 480,000 and degree of carboxymethylation is in a range of from 0.23 to 0.47.

11. A carboxymethyldextran polyalcohol which is used for preparation of the DDS compound according to any one of claims 1 to 6, wherein a weight-average molecular weight based on pullulan standard is in a range of from 240,000 to 480,000 and degree of carboxymethylation is in a range of from 0.14 to 0.47.

12. A carboxymethyldextran polyalcohol which is used for preparation of the DDS compound according to any one of claims 1 to 6, wherein a weight-average molecular weight based on pullulan standard is in a range of from 240,000 to 480,000 and degree of carboxymethylation is in a range of from 0.14 to 0.38.

13. The carboxymethyldextran polyalcohol according to any one of claims 10 to 12, wherein the degree of carboxymethylation is measured by capillary electrophoresis using a calibration curve which is obtained by measuring a carboxymethyldextran polyalcohol as a standard substance by decomposition method or NMR method.

14. The carboxymethyldextran polyalcohol according to claim 10, wherein the degree of carboxymethylation is measured by the capillary electrophoresis using a calibration curve which is obtained by measuring a carboxymethyldextran polyalcohol as a standard substance by the decomposition method.

15. The carboxymethyldextran polyalcohol according to claim 12, wherein the degree of carboxymethylation is measured by the capillary electrophoresis using a calibration curve which is obtained by measuring a carboxymethyldextran polyalcohol as a standard substance by the NMR method.

16. A method for preparing the carboxymethyldextran polyalcohol according to any one of claims 10 to 15, which comprises the steps of:
(A) adding an aqueous solution containing sodium periodate to an aqueous solution containing dextran at a temperature of 4°C ± 2°C to oxidize the dextran, and then adding the resulting reaction mixture to an aqueous solution containing sodium borohydride at a temperature not higher than 15°C to obtain a dextran polyalcohol; and
(B) reacting the dextran polyalcohol obtained in the step (A) with sodium monochloroacetate to prepare the carboxymethyldextran polyalcohol, **characterized in that** an end of carboxymethylation is determined by capillary electrophoresis.
